# EUROPÄISCHE PATENTSCHRIFT

(11) **EP 0 992 258 B1**
(45) Veröffentlichungstag und Bekanntmachung des Hinweises auf die Patenterteilung: **29.11.2006**
(21) Anmeldenummer: 99119458.0
(22) Anmeldetag: 30.09.1999
(51) Int. Cl.: A61M 5/315

(54) **Injektionsspritze**
Injection syringe
Seringue d'injection

(30) Priorität: 05.10.1998 DE 19845618
(43) Veröffentlichungstag der Anmeldung: 12.04.2000
(73) Patentinhaber: Linhardt GmbH & Co. KG, 94234 Viechtach (DE)
(72) Erfinder: Beil, Johann, D-93462 Lam (DE)
(74) Vertreter: Graf, Helmut

(56) Entgegenhaltungen:
- DE-U- 8 701 501
- US-A- 4 011 868
- US-A- 4 507 118
- US-A- 5 807 346

## Beschreibung

Die Erfindung bezieht sich auf eine Injektionsspritze gemäß Oberbegriff Patentanspruch 1.

Eine Injektionsspritze dieser Art zum dosierten Impfen bzw. Spritzen von Insulin ist bekannt (DE 87 01 501 U1). Bei dieser bekannten Injektionsspritze ist das Stößelelement vollständig in dem das erste Gehäuseteil umgebenden zweiten Gehäuseteil aufgenommen, sodass die Injektionsspritze auch vor dem eigentlichen Gebrauch eine relativ große Baulänge aufweist, und zwar insbesondere dann, wenn für die Vorratskammer ein großes Volumen erforderlich ist, wie dies insbesondere bei einem Einsatz einer Injektionsspritze im tiermedizinischen Bereich dann erforderlich ist, wenn in kurzer Zeit eine Vielzahl von Tieren geimpft werden müssen.

Nachteilig ist bei der bekannten Injektionsspritze auch, dass bei jedem nach einem Impfhub erforderlichen Rückhub ein Zurückbewegen des Kolbens mit dem zweiten Gehäuseteil nur dadurch verhindert ist, dass der Kolben im ersten Gehäuseteil mit einer gewissen Reibung verschiebbar geführt ist. Eine eindeutige Sperre des Kolbens beim Rückhub ist dadurch nicht gewährleistet.

Insbesondere in der Tiermedizin ist es aber vielfach erforderlich, eine Vielzahl von Tieren in möglichst kurzer Zeit mit jeweils einer vorgegebenen Menge eines Impfstoffes (Impf-Volumen) zu impfen.

Aufgabe der Erfindung ist es, eine Injektionsspritze aufzuzeigen, die dies bei preiswerter Ausführung rationell und zeitsparend ermöglicht. Zur Lösung dieser Aufgabe ist eine Injektionsspritze entsprechend dem Patentanspruch 1 ausgebildet.

Bei der erfindungsgemäßen Injektionsspritze wird das Stößelelement erst beim erstmaligen Gebrauch der Injektionsspritze von der der Injektionsnadel abgewandten Rückseite her durch die ersten und zweiten Rast- oder Klemmittel hindurch eingesetzt, sodass die Injektionsspritze für die Lieferung und Lagerung eine kurze Baulänge aufweist.

Bei der Erfindung ist der Impfstoff in einer im ersten Gehäuseteil vorgesehenen und als Zylinderkammer mit Kolben ausgebildeten Vorratskammer untergebracht, wobei am ersten Gehäuseteil auch die Injektionsnadel vorgesehen ist. Das zweite Gehäuseteil ist am ersten Gehäuseteil beweglich vorgesehen, und zwar für einen Impfhub aus einer Ausgangsstellung und für einen Rückhub in die Ausgangsstellung. Durch die Mitnahmemittel wird der Kolben bei jedem Impfhub mit dem zweiten Gehäuseteil im Sinne einer Verkleinerung des Volumens der Vorratskammer mitgeführt. Bei jedem Rückhub wird der Kolben von den Mitnahmemitteln freigegeben, so daß er mit dem zweiten Gehäuseteil nicht mitgeführt wird, sondern in seiner Stellung verbleibt, die er nach dem Impfhub eingenommen hat, so daß das Gesamtvolumen der Vorratskammer schrittweise von Impfvorgang zu Impfvorgang verkleinert wird.

Weiterbildungen der Erfindung sind Gegenstand der Unteransprüche. Die Erfindung wird im Folgenden anhand der Figuren an einem Ausführungsbeispiel näher erläutert. Es zeigen:
- Fig. 1: eine Mehrfach-Injektionsspritze gemäß der Erfindung in Seitenansicht;
- Fig. 2: eine Draufsicht auf die Rückseite der Injektionsspritze der Fig. 1;
- Fig. 3: einen Längsschnitt durch die Injektionsspritze der Fig. 1;
- Fig. 4: in Einzeldarstellung und teilweise geschnitten einen Dosierring der Injektionsspritze der Figuren 1 - 3;
- Fig. 5: in Teildarstellung und im Schnitt das äußere, kreiszylinderförmige Gehäuseteil der Injektionsspritze der Figuren 1 - 3;
- Fig. 6: in vergrößerter Teildarstellung eine Verzahnung einer Zahnstange.

Die in den Figuren allgemein mit 1 bezeichnete Injektionsspritze ist als Mehrfachspritze ausgebildet und insbesondere zur Verwendung in der Tiermedizin für das Impfen einer Vielzahl von Tieren, beispielsweise von Schweinen, Ferkeln oder Kälbern mit Antibiotika oder anderen Substanzen bestimmt.

Die generelle Funktionsweise ist derart, daß die Spritze 1 in einem in den Figuren allgemein mit 2 bezeichneten langgestreckten Spritzenkörper oder Gehäuse einen für eine Vielzahl von Impfungen ausreichenden Vorrat an Impfstoff enthält, daß beim Ansetzen der Injektionsspritze 1 mit einem in den Figuren kegelförmig ausgebildeten Ende 3 auf die Haut des zu impfenden Tieres und durch Andrücken der Injektionsspritze 1 mit der Spitze 3 an das Tier zunächst eine Injektionsnadel 4 durch eine Öffnung aus dem kegelförmigen Ende 3 vorbewegt und in das Gewebe des Tieres eingeführt wird, und daß dann anschließend eine vorgegebene Menge an Impfstoff aus dem Vorrat in das Tier injiziert wird. Nach Abschluß des Impfvorganges wird bei nicht mehr angedrückter Injektionsspritze u.a. auch die Injektionsnadel 4 wieder eingezogen. Die Injektionsspritze 1 ist dann für eine weitere Impfung bereit. Während jeder Impfung bildet die abgerundete Spitze 3 eine Anlage- oder Abstützfläche, mit der sich die Injektionsspritze 1 an dem zu impfenden Tier usw. abstützt.

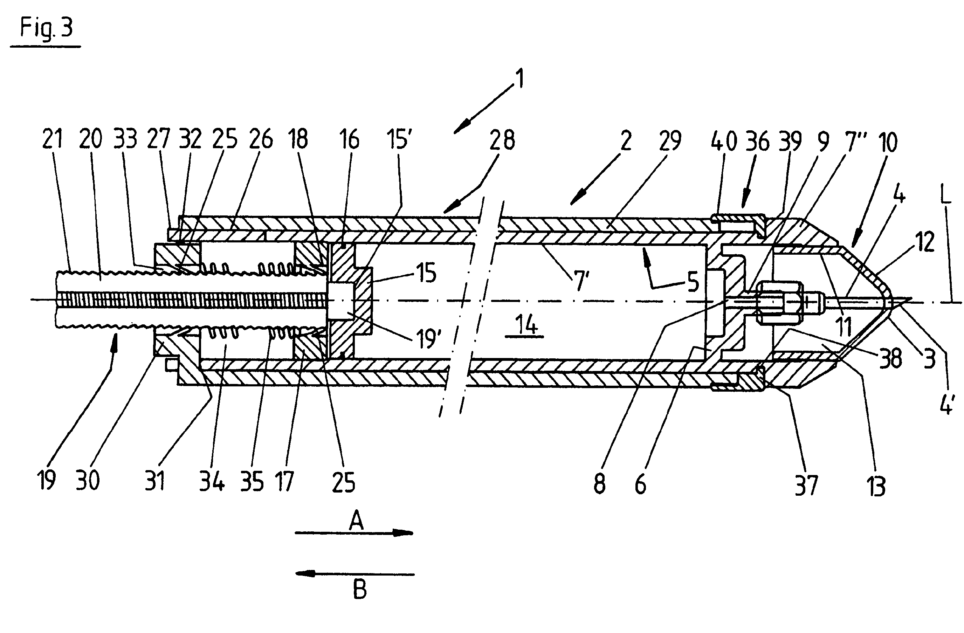

Für diese generelle Arbeitsweise besitzt die Injektionsspritze 1 den in den Figuren 3 - 5 mehr im Detail dargestellten Aufbau. Im einzelnen umfaßt das Gehäuse 2 u.a. ein inneres Gehäuseteil 5, welches im wesentlichen als Hohlzylinder mit kreisförmigem Innen- und Außendurchmesser und mit Zwischenboden 6 einstückig hergestellt ist, und zwar derart, daß die die Achse des Gehäuseteils 5 konzentrisch umschließende Umfangs- oder Zylinderwand von zwei Abschnitten 7' und 7'' gebildet ist, von denen sich der Abschnitt 7' bei der für die Fig. 3 gewählten Darstellung vom Zwischenboden 6 nach links und der Abschnitt 7'' vom Zwischenboden 6 nach rechts weg erstrecken. Mit seiner Achse liegt das Gehäuseteil 5 achsgleich mit der Längsachse L der Injektionsspritze 1. Im Zwischenboden 6 ist in der Mitte ein achsgleich mit der Längsachse L liegender Kanal 8 gebildet, der sich an dem vom Abschnitt 7'' gebildeten Teil des Gehäuseteils 5 in einem hohlzapfenartigen Vorsprung 9 fortsetzt, auf dem die Injektionsnadel 4 befestigt ist, und zwar derart, daß sich der Kanal 8 auch in den Injektionskanal der die Injektionsnadel 4 fortsetzt, die an ihrem Ende zur Bildung der Nadelspitze 4' in der üblichen Weise abgeschrägt ist.

In dem vom Abschnitt 7'' gebildeten offenen Ende des Gehäuseteils 5 ist ein Formteil oder eine Kappe 10 eingesetzt, welche im wesentlichen aus einem kreisring- oder kreiszylinderförmigen, die Längsachse L konzentrisch umschließenden Abschnitt 12 und einem sich an einer Seite anschließenden kegelförmigen Abschnitt 12 zusammensetzt, und welche in der vom Abschnitt 7'' gebildeten Öffnung des Gehäuseteils 5 gegen die Wirkung einer nicht dargestellten Druckfeder axial derart verschiebbar ist. Der Abschnitt 12 steht über die betreffende Seite des Gehäuseteils 5 und bildet damit die Spitze 3 oder Anlage- bzw. Abstützfläche der Injektionsspritze 1. Im nicht betätigten Zustand ist die Injektionsnadel 4 in dem von dem Abschnitt 7'' und dem Formteil 10 gebildeten Raum 13 aufgenommen, und zwar bei der in den Figuren dargestellten Ausführungsformen in der Weise, daß die Injektionsnadel 4 nur mit ihrer Spitze 4' geringfügig aus dem Formteil 10 bzw. über das spitze Ende 3 der Injektionsspritze 1 vorsteht.

Grundsätzlich ist es aber auch möglich, daß im Nicht-Gebrauchszustand die Injektionsnadel 4 einschließlich ihrer Spitze 4' vollständig in dem Raum 13 aufgenommen ist. Bevorzugt ist der Raum 13 mit einem weichen Trägermaterial, beispielsweise mit Watte für ein Desinfektions- oder Reinigungsmittel, beispielsweise für Alkohol gefüllt, so daß zumindest jeder Injektion die Nadel 4 gereinigt und desinfiziert wird. Das Gehäuseteil 5 bzw. der Abschnitt 7" der Umfangswand dieses Gehäuseteils ist in dem das Formteil 10 aufnehmenden Teilbereich mit einem verstärkten Wandabschnitt ausgebildet.

Der Abschnitt 7' der Umfangswand des Gehäuseteils 5 bildet die Umfangswand eines Zylinderraumes oder einer Kammer 14, in der der Vorrat in Injektionsmaterial aufgenommen ist. Dem Zwischenboden 6 gegenüberliegend ist die Kammer 14 durch einen Kolben 15 begrenzt, der über einen die Längsachse L konzentrisch umschließenden Dichtungsring 16 dicht gegen die Innenfläche des Abschnittes 7' an liegt.

In den Abschnitt 7' ist weiterhin eine Scheibe 17 eingesetzt, und zwar im Bereich des dem Zwischenboden 6 entfernt liegenden Endes des Abschnittes 7'. Die Scheibe 17 befindet sich an der dem Zwischenboden 6 abgewandten Seite des Kolbens 5 und dient u.a. als Anlage für den Kolben 15 bei dem größten, möglichen Volumen der Kammer 14. Die Scheibe 17 hat aber noch weitere, nachstehend näher beschriebene Funktionen. In der Scheibe 17 ist mittig eine Öffnung 18 vorgesehen, die in ihrem Querschnitt an den Querschnitt einer Zahnstange 19 angepaßt ist. Die Scheibe 17 ist im Inneren des Abschnitts 7' in geeigneter Weise fixiert, beispielsweise durch Kleben, Schweißen usw..

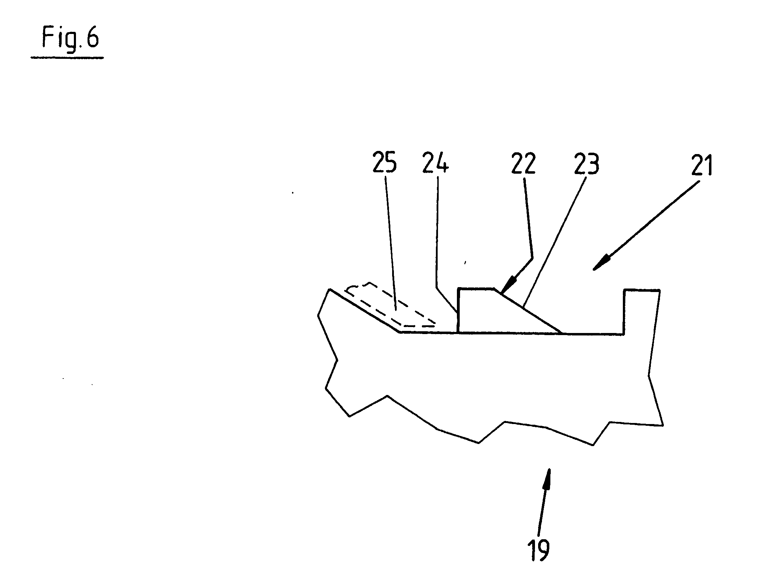

Bei der dargestellten Ausführungsform besitzt die Zahnstange 19 ein Ende 19' mit kreiszylinderförmigen Querschnitt sowie einen kreuzförmigen Querschnitt, der von vier, sich jeweils in Längsrichtung der Zahnstange 19 erstreckenden leistenartigen Querschnittsabschnitten 20 gebildet ist, die um die Längsachse der Zahnstange 19 um 90° gegeneinander versetzt und an ihren außenliegenden Rändern jeweils mit einer Verzahnung 21 versehen sind, deren Zähne 22 in der der Fig. 6 dargestellten Form sägezahnähnlich ausgeführt sind, und zwar mit einer schräg verlaufenden vorderen Flanke 23 an der dem Ende 19' zugewandten Seite jedes Zahnes 22 und einer im wesentlichen senkrecht zur Achse der Zahnstange verlaufenden rückwärtigen Flanke 24.

Die Scheibe 17 ist weiterhin im Bereich der Öffnung 18 mit vier federnden Rasten 25 versehen, die nach dem Einführen der Zahnstange 19 in die Öffnung 18 derart mit jeweils einer Verzahnung 21 zusammenwirken, daß die Rasten 25 ein Vorbewegen der Zahnstange 19 in der Längsachse L in Richtung auf den Zwischenboden 6 (Pfeil A der Fig. 3) zulassen, die Zahnstange 19 aber für eine Bewegung in umgekehrter Richtung (Pfeil B der Fig. 3) sperren. Die Öffnung 18 ist an den Querschnitt der Zahnstange 19 angepaßt und besitzt hierfür einen Querschnitt, der sich aus einem kreisförmigen Querschnitt (für das Ende 19') mit vier jeweils um 90° gegeneinander versetzten radialen Erweiterungen zusammensetzt, in denen jeweils Rast 25 angeordnet ist.

An der dem Kolben 15 abgewandten Seite der Scheibe 19 setzt sich die Umfangswand des Gehäuseteils 5 in mehreren, in gleichmäßigen Winkelabständen um die Längsachse L verteilten, mit ihrer Längserstreckung jeweils in Richtung bzw. parallel zur Längsachse L erstreckenden Stegen 26 fort, die hinsichtlich ihrer Formgebung und Anordnung so erscheinen, als ob das kreiszylinderförmige Gehäuseteil im Bereich dieser Stege 26 mit breiteren Schlitzen versehen ist, die von dem dortigen Ende ausgehen und die Stege 26 zwischen sich bilden. Jeder Steg besitzt an seinem freien Ende eine über die der Längsachse L abgewandte Außenfläche radial vorstehende, teilringartige Rast 27.

Das Gehäuse 2 besteht weiterhin aus einem Gehäuseteil 28 mit einer kreiszylinderförmigen Umfangswand 29, die das Gehäuseteil 5 an seiner Umfangswand 7'/7'' umschließt und axial, d.h. in Richtung der Längsachse L auf dem Gehäuseteil 5 verschiebbar angeordnet ist. An dem einen Ende ist das Gehäuseteil 28 mit einer Scheibe 30 versehen, die über radiale Stege 31 mit dem dortigen Ende der Umfangswand 29 verbunden ist. Die Stege 31 sind um die Längsachse L derart verteilt, daß zwischen zwei Stegen 31 jeweils eine Öffnung 32 gebildet ist, und zwar zwischen diesen Stegen und der Außenfläche der Scheibe 30 und der Innenfläche der Umfangswand 29. Durch jeweils eine Öffnung 32 reicht in Steg 26 hindurch, der dann an seinem aus der Öffnung 32 vorstehenden Ende mit dem Rastabschnitt 27 das Gehäuseteil 28 bzw. dessen Umfangswand 29 an dem der Scheibe 30 benachbarten Rand hintergreift, wodurch der Bewegungshub des Gehäuseteils 28 auf dem Gehäuseteil 5 bei der für die Figur gewählten Darstellung nach links, d.h. in Richtung des Pfeiles B in eine Ausgangsstellung begrenzt ist.

In der Scheibe 30 ist eine der Öffnung 18 entsprechende Öffnung 33 vorgesehen, die wiederum in ihrem Querschnitt dem Querschnitt der Zahnstange 19 angepaßt ist und innen für jede Verzahnung 21 mit einer Rast 25 versehen ist, die ein Vorschieben der Zahnstange in Richtung des Pfeiles A ermöglicht, die Zahnstange an der Scheibe 30 gegen eine Bewegung in umgekehrter Richtung, d.h. in Richtung des Pfeiles B sperrt.

Die Anordnung ist weiterhin so getroffen, daß die beiden Scheiben 17 und 30 in Richtung der Längsachse L einen Abstand aufweisen, der auf jeden Fall größer ist als der mögliche Bewegungshub des Gehäuseteils 28 relativ zum Gehäuseteil 5 aus der in der Figur 3 dargestellten Ausgangsstellung in Richtung des Pfeiles A. In dem zwischen den beiden Scheiben 17 und 30 gebildeten Raum 34 ist eine zwischen diesen Scheiben wirkende Druckfeder 35 angeordnet, die beim Einsatz der Injektionsspritze 1 die Zahnstange 19 umschließt und das Gehäuseteil 28 in die Ausgangsstellung vorspannt, in der dieses Gehäuseteil gegen die Rasten 27 anliegt.

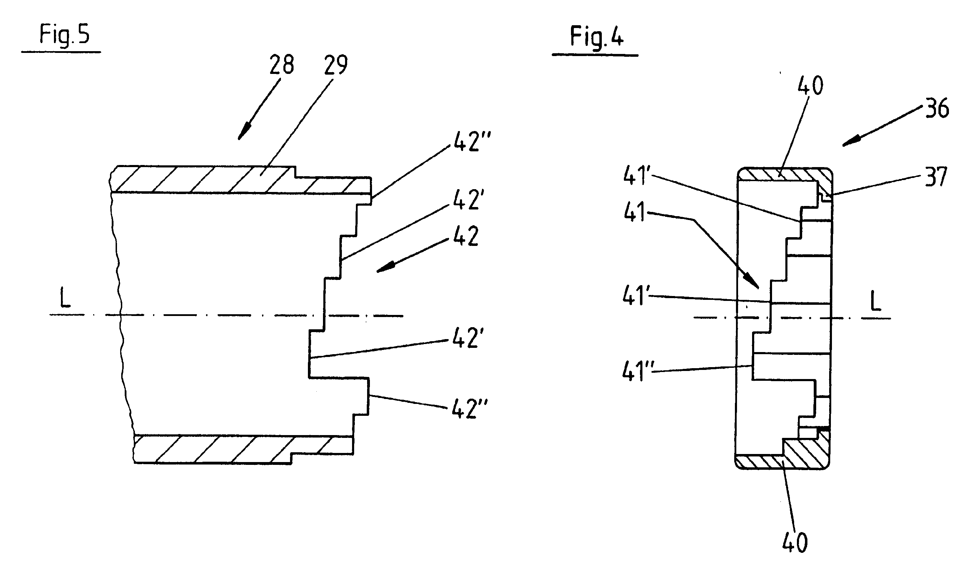

Mit 36 ist ein Dosierring bezeichnet, der an der Außenfläche des Gehäuseteil 5 bzw. des Wandabschnittes 7'' um die Längsachse L drehbar vorgesehen ist. Der Dosierring 36 greift hierfür mit einem Ringabschnitt 37 in eine die Längsachse L konzentrisch umschließende Ringnut 38 und stützt sich mit diesem Ringabschnitt an einem der Ringnut 38 benachbarten und durch die vorgenannte Verdickung des Abschnittes 7" gebildeten ringförmigen Bund 39 des Gehäuseteils 5 axial ab. Ausgehend von dem Ringabschnitt 37 ist der Dosierring 36 weiterhin mit einem kreiszylinderförmigen, die Längsachse L konzentrisch umschließenden Wandabschnitt 40 ausgebildet, der bei der dargestellten Ausführungsform an seiner Innenfläche vier in Umfangsrichtung aufeinanderfolgende, jeweils gleichgeformte Kulissenabschnitten 41 bildet. Jeder Kulissenabschnitt 41 ist so ausgeführt, daß er Stufen 41' bildet, deren axialer Abstand von dem Ringabschnitt 37 bzw. dem Bund 39 in einer angenommenen Umfangsrichtung ausgehend von einem kleinsten Abstand stufenförmig zunimmt und dann am Ende des jeweiligen Kulissenabschnittes 41 wieder auf den kürzesten axialen Abstand zurückspringt, wie dies in der Fig. 4 dargestellt ist.

Das der Scheibe 30 entfernt liegende und dem Dosierring 36 benachbarte Ende des Wandabschnittes 29 ist mit den Kulissenabschnitten 41 konformen Kulissenabschnitten 42 versehen. Diese sind entsprechend der Fig. 5 so ausgeführt, daß jeder Kulissenabschnitt 42 Stufen 42' aufweist, die mit unterschiedlicher sich treppen- oder stufenartig ändernder axialer Länge über das dortige Ende des Gehäuseteils 28 vorstehen. Die Stufen 42' sind gleich den Stufen 41', wobei aber die Stufen 42' in einer Umfangsrichtung ansteigen, die derjenigen Umfangsrichtung entgegengesetzt ist, in der die Stufen 41' ansteigen. Durch die zusammenwirkenden Kulissenabschnitte 41 und 42 ist der maximale Hub des Gehäuseteils 28 relativ zum Gehäuseteil 5 in Richtung des Pfeiles A einstellbar, und zwar in Abhängigkeit von der Stellung des Dosierringes 36. Durch die stufenförmige Ausbildung der Kulissen 41 und 42 ist gewährleistet, daß am Ende jedes Impfhubes dieser jeweils durch das Anliegen einer Vielzahl von Flächen der Kulissen 41 und 42 begrenzt wird, die bei Beendigung der Bewegung des Gehäuseteils 28 relativ zum Gehäuseteil 5 auftretenden Anschlagkräfte also großflächig vom Gehäuseteil 28 über den Dosierring 26 auf das Gehäuseteil 5 übertragen werden.

Bevorzugt sind die Kulissen 41 und 42 so ausgebildet, daß deren Abschnitte oder Stufen 41" und 42" mit der größten axialen Stufenhöhe den Winkel begrenzen, um den der Dosierring 36 zum Einstellen gedreht werden kann, d.h. in beiden Endstellungen liegt dann der Abschnitt 41'' jeder Kulisse 41 jeweils gegen eine Seite eines Abschnittes 42'' der Kulisse 42 an.

Bevorzugt ist der Dosierring 36 an seiner Außenfläche mit einer Rändelung 43 und beispielsweise auch mit einer Markierung 44 versehen, der eine Skala 45 am äußeren Gehäuseteil 28 zugeordnet ist.

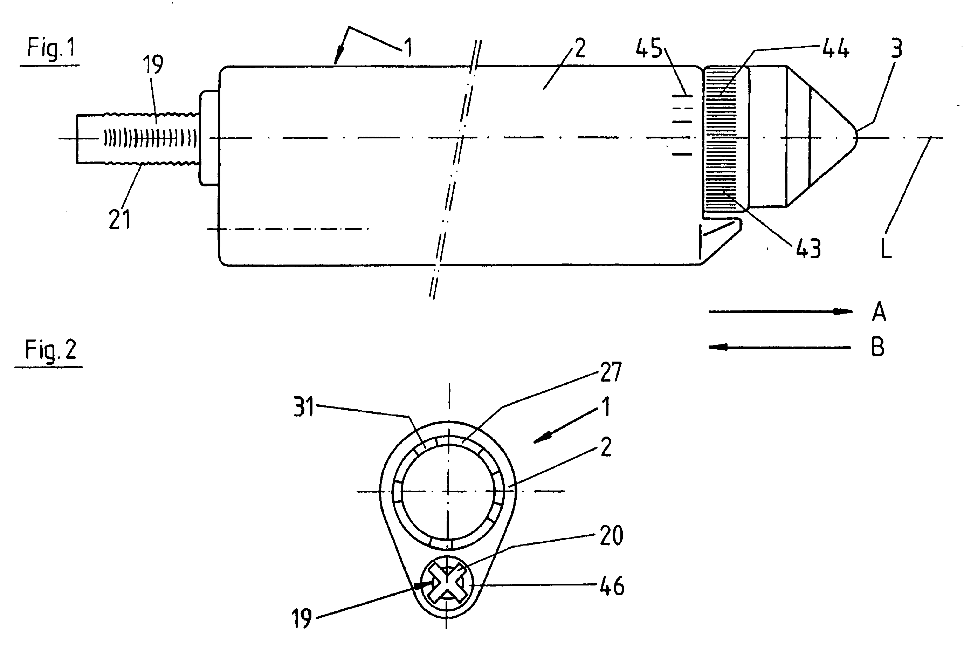

Bei der in den Figuren 1 und 2 dargestellten Ausführungsform ist das Gehäuse 2 bzw. das äußere Gehäuseteil 29 noch mit einer Ausnehmung 46 versehen, die mit ihrer Achse parallel zur Längsachse L liegt, gegenüber dieser allerdings radial versetzt ist. Die Ausnehmung 46 dient zur Aufnahme der Zahnstange 19 während des Transportes und der Lagerung der Injektionsspritze 1. Bei der dargestellten Ausführungsform ist die Zahnstange 19 dann nahezu vollständig von der Ausnehmung 46 aufgenommen. Für den Gebrauch wird die Zahnstange 19 der Ausnehmung 46 entnommen und an der der Spitze 3 abgewandten Rückseite in die Injektionsspritze 1 eingesetzt, und zwar mit ihrem vorderen Ende 19' voraus durch die Öffnungen 33 und 18 der Scheiben 30 bzw. 17 in eine dem Ende 19' angepaßte Vertiefung 15' des Kolbens 15.

Durch die Rasten 25 ist die Zahnstange in den Öffnungen 18 und 33 jeweils gegen Zurückbewegen in Richtung des Pfeiles B gesichert. Für den Impfvorgang wird die Injektionsspritze 1 am äußeren Gehäuseteil 28 außen gegriffen. Nach dem Aufsetzen des Endes 3 auf die Haut des zu impfenden Tieres wird durch Steigerung des Anpreßdruckes zunächst die Kappe 10 zurückgeschoben, so daß die Nadel 4 in das Gewebe eindringt. Sobald die Kappe 10 gegen die Zwischenwand 6 anliegt, wird das äußere Gehäuseteil 28 entgegen der Wirkung der Druckfeder 35 relativ zum inneren Gehäuseteil 5 in Richtung des Pfeiles A bewegt, wobei die in den Rasten 25 der Scheibe 30 eingerastete Zahnstange 29 der Kolben 15 mitbewegt, so daß eine dem Impfhub des äußeren Gehäuseteils 28 entsprechende Menge an Injektionsmaterial aus der Kammer 14 über die Nadel 4 injiziert wird.

Nach dem Abnehmen der Injektionsspritze 1 kehrt das äußere Gehäuseteil 28 durch die Wirkung der Druckfeder 35 in seine Ausgangsstellung zurück, in der das innere Gehäuseteil mit den Rasten 27 am äußeren Gehäuseteil 28 anliegt. Bei diesem Rückhub ist der Kolben 15 durch die in der Scheibe 17 durch die dortigen Rasten 25 verriegelte Zahnstange 19 relativ zu dieser Scheibe und damit relativ zu dem inneren Gehäuseteil 5 verriegelt. Bei dem Rückhub ist eine durch die Rasten 25 unbehinderte Bewegung der Scheibe 30 auf der Zahnstange 19 in Richtung des Pfeiles B möglich, die (Zahnstange 19) bei jedem Impfvorgang zunehmend tiefer in das Gehäuse 2 hineinbewegt wird..

Die Gehäuseteile 5 und 28, das Formteil 10, der Kolben 15, die Scheiben 17 und 30 sowie die Zahnstange 19 sind jeweils als Formteile aus Kunststoff gefertigt, wobei die Rasten 25 einstückig mit den Scheiben 17 und 30 hergestellt sind. Ebenso ist auch der Dosierring 36 aus Kunststoff gefertigt.

Die beschriebene Injektionsspritze 1 ist für mehrere, jeweils dosierte Injektionen bestimmt, allerdings als Einwegspritze insofern, als nach dem Verbrauch des Injektionsmaterials in der Kammer 14 die Spritze entsorgt wird.

### Bezugszeichenliste

- 1: Injektionsspritze
- 2: Spritzengehäuse
- 3: Ende
- 4: Injektionsnadel
- 4': Spitze
- 5: inneres Gehäuseteil
- 6: Zwischenboden
- 7', 7": Umfangswand-Abschnitt
- 8: Kanal
- 9: Vorsprung
- 10: Formteil
- 11, 12: Abschnitt
- 13: Raum
- 14: Kammer
- 15: Kolben
- 15': Vertiefung
- 16: Kolbendichtung
- 17: Scheibe
- 18: Scheibenöffnung
- 19: Zahnstange
- 19': Ende
- 20: Querschnittsabschnitt
- 21: Verzahnung
- 22: Zahn
- 23, 24: Flanke
- 25: Rast
- 26: Steg
- 27: Rast
- 28: äußeres Gehäuseteil
- 29: Umfangswand
- 30: Scheibe
- 31: Steg
- 32: Öffnung
- 33: Scheibenöffnung
- 34: Raum
- 35: Druckfeder
- 36: Dosierring
- 37: Ringabschnitt
- 38: Ringnut
- 39: Bund
- 40: Wandabschnitt
- 41, 42: Kulisse
- 41', 42': Stufe
- 41", 42": Kulissenabschnitt oder Stufe
- 43: Rändelung
- 44: Markierung
- 45: Skala
- 46: Ausnehmung

## Patentansprüche

1. Injektionsspritze, insbesondere für den tiermedizinischen Bereich,
mit wenigstens einer Vorratskammer (14) in einem Gehäuse (2) zur Aufnahme des Impfstoffes, wobei die Kammer (14) durch wenigstens einen verschiebbaren Kolben (15) in ihrem Volumen veränderbar ist und über einen Kanal (8) mit dem Kanal einer an einem Ende (3) des Gehäuses vorgesehenen Injektionsnadel (4) in Verbindung steht, wobei für eine Mehrfachimpfung mit jeweils einem vorgegebenen Impf-Volumen, welches einen Bruchteil des maximalen Volumens der Vorratskammer (14) ausmacht, die Vorratskammer (14) und die Injektionsnadel (4) an einem ersten Gehäuseteil (5) vorgesehen sind, an welchem ein zweites, die Grifffläche der Injektionsspritze bildendes Gehäuseteil (28) für einen vorgegebenen Impfhub in einer Richtung (A) und für einen Rückhub in entgegengesetzter Richtung (B) bewegbar ist,
mit einem am Kolben (15) kolbenstangenartig angreifenden Stößelelement (19), mit einem ersten Rast- und Klemmelement (30) am zweiten Gehäuseteil (28), welches bei jedem Impfhub eine antriebsmäßige Verbindung des Stößelelementes (19) mit dem zweiten Gehäuseteil (28) für eine Bewegung des Kolbens (15) im Sinne einer Verkleinerung des Volumens der Vorratskammer (14) um das Impf-Volumen herstellt, sowie
mit Sperrmitteln (17, 19, 25), die bei jedem Rückhub ein Zurückbewegen des Kolbens (15) mit dem zweiten Gehäuseteil (28) verhindern,
**dadurch gekennzeichnet,**
**dass** die Sperrmittel von einem mit dem Stößelelement (19) zusammenwirkenden zweiten Rast- oder Klemmelement (17) am ersten Gehäuseteil (5) gebildet sind, und
**dass** das Stößelelement (19) für den ersten Gebrauch der Injektionsspritze (1) von der der Injektionsnadel (4) entfernt liegenden Rückseite der Injektionsspritze (1) her durch die ersten und zweiten Rast- oder Klemmelemente (30, 17) einsetzbar ist.

2. Injektionsspritze nach Anspruch 1, **gekennzeichnet durch** eine Aufnahme (46) zur Unterbringung des Stößelelementes (19) bei Nicht-Gebrauch, insbesondere bei dem Versand und der Lagerung.

3. Injektionsspritze nach Anspruch 1 oder 2, **dadurch gekennzeichnet, daß** am ersten Gehäuseteil (5) eine verschiebbare Kappe (10) vorgesehen ist, die in einer Ausgangsstellung die Injektionsnadel (4) zumindest teilweise abdeckt und beim Impfen in eine die Injektionsnadel (4) freigebende Stellung bewegbar ist.

4. Injektionsspritze nach einem der vorhergehenden Ansprüche, **dadurch gekennzeichnet, daß** das Stößelelement (19) sich von der der Vorratskammer (14) abgewandten Seite des Kolbens (15) weg erstreckt.

5. Injektionsspritze nach einem der vorhergehenden Ansprüche, **dadurch gekennzeichnet, daß** das erste Rast- oder Klemmelement (30) eine Klemmeinrichtung ist, die eine Bewegung des zweiten Gehäuseteils (28) relativ zum Stößelelement (19) in Richtung des Rückhubes zuläßt, das zweiten Gehäuseteil (28) aber für eine Bewegung in umgekehrter Richtung am Stößelelement (19) verriegelt.

6. Injektionsspritze nach einem der vorhergehenden Ansprüche, **dadurch gekennzeichnet, daß** das zweite Rast- oder Klemmelement (17) eine Klemmeinrichtung ist, die eine Bewegung des Stößelelementes (19) in Richtung einer Verkleinerung des Volumens der Vorratskammer (14) zuläßt, das Stößelelement (19) aber für eine Bewegung in umgekehrter Richtung am ersten Gehäuseteil (5) verriegelt.

7. Injektionsspritze nach einem der vorhergehenden Ansprüche, **dadurch gekennzeichnet, daß** das Stößelelement zahnstangenartig mit einer Vielzahl von Rastzähnen (22) ausgebildet ist, die wenigstens eine Zahnreihe (21) bilden, und daß das zweite Rastelement (30) am zweiten Gehäuseteil (28) und/oder das erste Rastelement (17) am ersten Gehäuseteil (5) wenigstens eine mit den Rastzähnen (22) zusammenwirkende Rast (25) aufweist.

8. Injektionsspritze nach einem der vorhergehenden Ansprüche, **dadurch gekennzeichnet, daß** das erste Gehäuseteil (5) am zweiten Gehäuseteil (28) in einer Achsrichtung parallel oder achsgleich mit der Achse der Injektionsnadel (4) verschiebbar vorgesehen ist.

9. Injektionsspritze nach einem der vorhergehenden Ansprüche, **dadurch gekennzeichnet, daß** das zweite Gehäuseteil (28) auf dem ersten Gehäuseteil (5) verschiebbar ist und eine Öffnung aufweist, welche das erste Gehäuseteil (5) zumindest teilweise aufnimmt.

10. Injektionsspritze nach einem der vorhergehenden Ansprüche, **dadurch gekennzeichnet, daß** das erste und/oder zweite Rast- oder Klemmelement jeweils von einem mit dem zweiten bzw. ersten Gehäuseteil (28, 5) verbundenen oder einstückig mit diesem Gehäuseteil hergestellten Gerhäuseabschnitt (30, 17) mit einer eine Rast oder Klemmeinrichtung für das Stößelelement (19) bildenden Öffnung (33, 18) gebildet sind.

11. Injektionsspritze nach einem der vorhergehenden Ansprüche, **gekennzeichnet durch** ein Dosierelement (36), welches beweglich am ersten Gehäuseteil (5) oder am zweiten Gehäuseteil (28) vorgesehen ist und einen einstellbaren, den Impfhub begrenzenden Anschlag bildet,
wobei beispielsweise das Dosierelement ein Dosierring (36) mit wenigstens einer unterschiedliche Anschläge für unterschiedliche Impfhübe bildenden Dosier-Kulisse (41) ist.

## Claims

1. Hypodermic syringe, more particularly for use in veterinary medicine,
with at least one supply chamber (14) in a housing (2) for holding the vaccine wherein the chamber (1) can be changed in its volume through at least one displaceable piston (15) and is connected through a channel (8) to the channel of a hypodermic needle (4) provided at one end (3) of the housing wherein for multiple vaccinations each time with a predetermined vaccine volume which makes up a fraction of the maximum volume of the supply chamber (14), the supply chamber (14) and the hypodermic needle (4) are provided on a first housing part (5) on which a second housing part (28) forming the grip face of the hypodermic syringe can move in one direction (A) for a predetermined vaccine stroke and in the opposite direction (B) for a return stroke,
with a plunger element (19) engaging in the manner of a piston rod on the piston (15), with a first detent and clamping element (30) on the second housing part (28) which with each vaccination stroke produces a drive-wise connection between the plunger element (19) and
the second housing part (28) for moving the piston (15) in the sense of reducing the volume of the supply chamber (14) by the vaccine volume,
as well as with locking means (17, 19, 25) which during each return stroke prevent the piston (15) from moving back with the second housing part (28),
**characterised in that** the locking means are formed by a second detent or clamping element (17) on the first housing part (5) interacting with the plunger element (19), and that the plunger element (19) for the first use of the hypodermic syringe (1) can be inserted from the back of the hypodermic syringe (1) lying remote from the hypodermic needle (4) through the first and second detent or clamping elements (30, 17).

2. Hypodermic syringe according to claim 1 **characterised by** a holder (46) for storing the plunger element (19) when not in use, more particularly during shipment and storage.

3. Hypodermic syringe according to claim 1 or 2 **characterised in that** on the first housing part (5) there is a displaceable cap (10) which in an initial position at least partially covers the hypodermic needle (4) and during vaccination can be moved into a position releasing the hypodermic needle (4).

4. Hypodermic syringe according to one of the preceding claims **characterised in that** the plunger element (19) extends away from the side of the piston (15) remote from the supply chamber (14).

5. Hypodermic syringe according to one of the preceding claims **characterised in that** the first detent or clamping element (30) is a clamping device which permits movement of the second housing part (28) relative to the plunger element (19) in the direction of the return stroke but locks the second housing part (28) for movement in the reverse direction on the plunger element (19).

6. Hypodermic syringe according to one of the preceding claims **characterised in that** the second detent or clamping element (17) is a clamping device which permits movement of the plunger element (19) in the direction of reducing the volume of the supply chamber (14), but locks the plunger element (19) for movement in the reverse direction on the first housing part (5).

7. Hypodermic syringe according to one of the preceding claims **characterised in that** the plunger element is designed like a toothed rod with several detent teeth (22) which form at least one row (21) of teeth, and that the second detent element (30) on the second housing part (28) and/or the first detent element (17) on the first housing part (5) has at least one catch (25) interacting with the detent teeth (22).

8. Hypodermic syringe according to one of the preceding claims **characterised in that** the first housing part (5) is provided displaceable on the second housing part (28) in an axial direction parallel to or coaxial with the axis of the hypodermic needle (4).

9. Hypodermic syringe according to one of the preceding claims **characterised in that** the second housing part (28) is displaceable on the first housing part (5) and has an opening which holds the first housing part (5) at least in part.

10. Hypodermic syringe according to one of the preceding claims **characterised in that** the first and/or second detent or clamping element are each formed by a housing section (30, 17) connected to the second or first housing part (28,5) or made integral with this housing part and having an opening (33, 18) forming a detent or clamping device for the plunger element (19).

11. Hypodermic syringe according to one of the preceding claims **characterised by** a dosing element (36) which is provided movable on the first housing part (5) or on the second housing part (28) and which forms an adjustable stop limiting the vaccination stroke, wherein by way of example the dosing element is a dosing ring (36) with at least one dosing slide guide (41) forming different stops for different vaccination strokes.

## Revendications

1. Seringue d'injection, en particulier pour le domaine de la médecine vétérinaire,
avec au moins une chambre de stockage (14) dans un boîtier (2) pour la réception de la substance d'inoculation, dans laquelle la chambre (14) est modifiable en volume par au moins un piston (15) coulissant et est en communication par l'intermédiaire d'un canal (8) avec le canal d'une aiguille d'injection (4) prévue à une extrémité (3) du boîtier, dans laquelle pour une inoculation multiple avec chaque fois un volume d'inoculation prédéfini qui représente une fraction du volume maximale de la chambre de stockage (14), la chambre de stockage (14) et l'aiguille d'injection (4) sont prévues sur une première partie (5) du boîtier sur laquelle une deuxième partie (28) du boîtier formant la surface de préhension de la seringue d'injection peut être déplacée pour une première course d'inoculation prédéfinie dans une direction (A) et pour une course de retour en direction opposée (B),
avec un élément pousseur (19) s'appliquant à la manière d'une tige de piston sur le piston (15),
avec un premier élément d'arrêt et de serrage (30) sur la second partie (28) du boîtier qui, lors de chaque course d'inoculation, établit une connexion d'entraînement de l'élément pousseur (19) avec la deuxième partie (28) du boîtier pour un mouvement du piston (15) dans le sens d'une diminution du volume de la chambre de stockage (14) de l'ordre du volume d'inoculation, ainsi que
avec des moyens de blocage (17, 19, 25) qui empêchent, lors de chaque course de retour, un déplacement vers l'arrière du piston (15) avec la deuxième partie (28) du boîtier,
**caractérisé**
**en ce que** les moyens de blocage sont formés par un deuxième élément d'arrêt et de serrage (17) sur la première partie (5) du boîtier qui coopère avec l'élément pousseur (19), et
**en ce que** l'élément pousseur (19) peut être inséré pour la première utilisation de la seringue d'injection (1) par le côté arrière de la seringue d'injection (1) qui est éloigné de l'aiguille d'injection (4), à travers les premier et deuxième éléments d'arrêt et de serrage (30, 17).'

2. Seringue d'injection selon la revendication 1, **caractérisée par** un logement (46) pour caser l'élément pousseur (19) en cas de non-utilisation, en particulier lors de l'expédition et de l'entreposage.

3. Seringue d'injection selon la revendication 1 ou 2, **caractérisée en ce qu'**il est prévu, sur la première partie (5) du boîtier, un cache (10) coulissant qui, dans une position de départ, recouvre au moins en partie l'aiguille d'injection (4) et qui, lors de l'inoculation, peut être déplacée dans une position libérant l'aiguille d'injection (4).

4. Seringue d'injection selon une des revendications précédentes, **caractérisée en ce que** l'élément pousseur (19) s'étend en s'éloignant du côté du piston (15) opposé à la chambre de stockage (14).

5. Seringue d'injection selon une des revendications précédentes, **caractérisée en ce que** le premier élément d'arrêt et de serrage (30) est un dispositif de serrage qui permet un déplacement de la deuxième partie (28) du boîtier par rapport à l'élément pousseur (19) dans le sens de la course de retour mais qui verrouille la deuxième partie (28) du boîtier pour un déplacement dans le sens inverse sur l'élément pousseur (19).

6. Seringue d'injection selon une des revendications précédentes, **caractérisée en ce que** le deuxième élément d'arrêt et de serrage (17) est un dispositif de serrage qui permet un déplacement de l'élément pousseur (19) dans le sens d'une diminution du volume de la chambre de stockage (14) mais qui verrouille l'élément pousseur (19) pour un déplacement dans le sens inverse sur la première partie (5) du boîtier.

7. Seringue d'injection selon une des revendications précédentes, **caractérisée en ce que** l'élément pousseur est conformé à la manière d'une crémaillère avec une pluralité de dents d'arrêt (22) qui forment au moins une rangée de dents (21), et **en ce que** le deuxième élément d'arrêt (30) sur la deuxième partie du boîtier (28) et/ou le premier élément d'arrêt (17) sur la première partie (5) du boîtier présente au moins un cran d'arrêt (25) coopérant avec les dents d'arrêt (22).

8. Seringue d'injection selon une des revendications précédentes, **caractérisée en ce que** la première partie (5) du boîtier est prévue de manière à coulisser sur la deuxième partie (28) du boîtier dans une direction axiale parallèle ou coïncidant avec l'axe de l'aiguille d'injection (4).

9. Seringue d'injection selon une des revendications précédentes, **caractérisée en ce que** la deuxième partie (28) du boîtier peut coulisser sur la première partie (5) du boîtier et présente une ouverture qui accueille au moins en partie la première partie (5) du boîtier.

10. Seringue d'injection selon une des revendications précédentes, **caractérisée en ce que** le premier et / ou le deuxième élément d'arrêt et de serrage sont formés respectivement par une section (30, 17) de boîtier reliée à la deuxième ou encore à la première partie (28, 5) du boîtier ou fabriquée d'un seul tenant avec cette partie du boîtier et comprenant une ouverture (33, 18) formant un cran d'arrêt ou un dispositif de serrage pour l'élément pousseur (19).

11. Seringue d'injection selon une des revendications précédentes, **caractérisée par** un élément doseur (36) qui est prévu de manière mobile sur la première partie (5) du boîtier ou sur la deuxième partie (28) du boîtier et forme une butée ajustable limitant la course d'inoculation,
dans laquelle l'élément doseur est, par exemple, un anneau doseur (36) avec au moins une coulisse de dosage (41) formant différentes butées pour différentes courses d'inoculation.
